(19) Europäisches Patentamt
European Patent Office
Office européen des brevets

(11) Veröffentlichungsnummer: **0 626 382 A1**

# (12) EUROPÄISCHE PATENTANMELDUNG

(21) Anmeldenummer: **94107474.2**

(22) Anmeldetag: **13.05.94**

(51) Int. Cl.5: **C07D 403/10**, C07D 403/12, A61K 31/415, A61K 31/41, A61K 31/40

(30) Priorität: **25.05.93 DE 4317321**

(43) Veröffentlichungstag der Anmeldung: **30.11.94 Patentblatt 94/48**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Krämer, Thomas, Dr.**
**Schneewittchenweg 37**
**D-42111 Wuppertal (DE)**
Erfinder: **Hanko, Rudolf, Dr.**
**Waldsaum 25**
**D-45134 Essen (DE)**
Erfinder: **Dressel, Jürgen, Dr.**
**Tuchstrasse 48**
**D-42477 Radevormwald (DE)**
Erfinder: **Fey, Peter, Dr.**
**Am Eickhof 23**
**D-42111 Wuppertal (DE)**
Erfinder: **Hübsch, Walter, Dr.**
**Wildsteig 22**
**D-42113 Wuppertal (DE)**
Erfinder: **Müller, Ulrich E., Dr.**
**Neuer Triebel 91**
**D-42111 Wuppertal (DE)**
Erfinder: **Müller-Gliemann, Matthias, Dr.**
**Laibacher Strasse 10**
**D-42697 Solingen (DE)**
Erfinder: **Beuck, Martin, Dr.**
**Trills 7**
**D-40699 Erkrath (DE)**
Erfinder: **Bischoff, Hilmar, Dr.**
**Am Rohm 78**
**D-42113 Wuppertal (DE)**
Erfinder: **Wohlfeil, Stefan, Dr.**
**Tucherweg 25**
**D-40724 Hilden (DE)**
Erfinder: **Denzer, Dirk, Dr.**
**Claudiusweg 7**
**D-42115 Wuppertal (DE)**
Erfinder: **Kazda, Stanislav, Prof. Dr.**
**Gellertweg 18**
**D-42115 Wuppertal (DE)**
Erfinder: **Stasch, Johannes-Peter, Dr.**
**Alfred-Nobel-Strasse 109**
**D-42651 Solingen (DE)**
Erfinder: **Knorr, Andreas, Dr.**
**Trillser Graben 10**
**D-40699 Erkrath (DE)**
Erfinder: **Zaiss, Siegfried, Dr.**
**Farnweg 3**
**D-42113 Wuppertal (DE)**

(54) **Substituierte 2,4-Imidazolidindione als blutdrucksenkende und anti-atherisklerotische Mittel.**

(57) Substituierte 2,4-Imidazolidindione der allgemeinen Formel

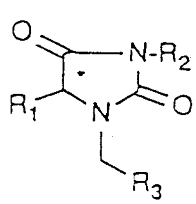

(I)

EP 0 626 382 A1

Rank Xerox (UK) Business Services
(3.10/3.09/3.3.4)

in welcher

- $R^1$ für geradkettiges oder verzweigtes Alkyl mit bis zu 8 Kohlenstoffatomen steht,
- $R^2$ für Cycloalkyl mit 3 bis 8 Kohlenstoffatomen steht, oder für geradkettiges oder verzweigtes Alkyl mit bis zu 8 Kohlenstoffatomen steht, das gegebenenfalls durch Phenyl substituiert ist, oder für eine Gruppe der Formel $-CH_2-CO_2R^4$ steht,
- $R^3$ für einen Rest der Formel

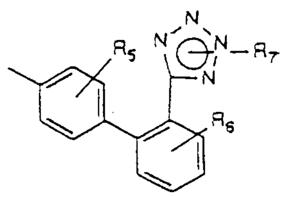

oder

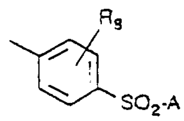

steht,

werden hergestellt entweder durch Umsetzung von α-Aminocarbonsäurederivaten mit entsprechend substituierten Biphenylmethylhalogeniden und anschließender Cyclisierung mit Isocyanat oder durch Umsetzung von N-benzylsubstituierten α-Aminocarbonsäurederivaten mit Tetrazolylphenylboronsäuren oder durch Umsetzung von sulfonylsubstituierten Benzylhalogeniden mit α-Aminocarbonsäurederivaten und anschließende Cyclisierung mit Isocyanaten. Die erfindungsgemäßen Verbindungen können als Wirkstoffe in Arzneimitteln eingesetzt werden, bevorzugt zur Behandlung von arterieller Hypertonie sowie Arteriosklerose.

Die Erfindung betrifft substituierte 2,4-Imidazolidindione, Verfahren zu ihrer Herstellung sowie ihre Verwendung in Arzneimitteln, insbesondere als blutdrucksenkende und anti-atherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Dartiber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Phenylmethyl- und Biphenylmethyl-Hydantoin-Derivate sind bereits aus den Publikationen JP 52/100 469 und JP 52/ 140 470 bekannt.

Die vorliegende Erfindung betrifft substituierte 2,4-Imidazolidindione der allgemeinen Formel (I)

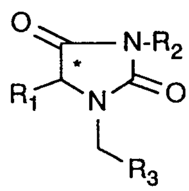

(I)

in welcher

$R^1$ für geradkettiges oder verzweigtes Alkyl mit bis zu 8 Kohlenstoffatomen steht,

$R^2$ für Cycloalkyl mit 3 bis 8 Kohlenstoffatomen steht, oder
für geradkettiges oder verzweigtes Alkyl mit bis zu 8 Kohlenstoffatomen steht, das gegebenenfalls durch Phenyl substituiert ist, oder
für eine Gruppe der Formel $-CH_2-CO_2R^4$ steht,
worin

$R^4$ Wasserstoff oder geradkettiges oder verzweigtes Alkyl mit bis zu 8 Kohlenstoffatomen bedeutet,
oder

für Phenyl steht, das gegebenenfalls durch Halogen, Cyano, Trifluormethyl oder Trifluormethoxy substituiert ist,

$R^3$ für einen Rest der Formel

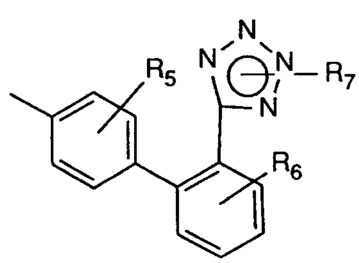

oder

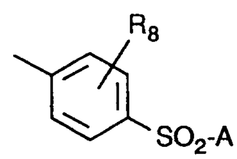

steht,
worin

$R^5$, $R^6$ und $R^8$ gleich oder verschieden sind und
Wasserstoff, Halogen, Cyano, Nitro oder für geradkettiges oder verzweigtes Alkyl oder Alkoxy mit jeweils bis zu 6 Kohlenstoffatomen bedeuten,

$R^7$ Wasserstoff, geradkettiges oder verzweigtes Alkyl mit bis zu 6 Kohlenstoffatomen oder die Triphenylmethylgruppe bedeutet,

A einen über das Stickstoffatom gebundenen 3 bis 8-gliedrigen, gesättigten Heterocyclus mit bis zu 2 Heteroatomen aus der Reihe S, N oder O bedeutet und der gegebenenfalls bis zu 2-fach gleich oder verschieden durch einen Rest der Formel

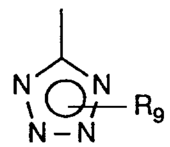

oder -CO-$R^{10}$ substituiert ist,
worin

$R^9$ die oben angegebene Bedeutung von $R^7$ hat und mit dieser gleich oder verschieden ist,

$R^{10}$ Hydroxy oder geradkettiges oder verzweigtes Alkoxy mit bis zu 6 Kohlenstoffatomen bedeutet, oder
eine Gruppe der Formel $-NR^{11}R^{12}$ bedeutet,
worin

$R^{11}$ und $R^{12}$ gleich oder verschieden sind und Wasserstoff oder geradkettiges oder verzweigtes Alkyl mit bis zu 6 Kohlenstoffatomen bedeuten

und deren Salze.

Die erfindungsgemäßen substituierten 2,4-Imidazolidindione können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der substituierten 2,4-Imidazolidindione können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure, Trifluoressigsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium-oder Calciumsalze, sowie Ammoniumsalze, die abgeleitet sind von Ammoniak oder organischen Aminen wie beispielsweise Ethylamin, Di- bzw. Triethylamin, Di-bzw. Triethanolamin, Dicyclohexylamin, Dimethylaminoethanol, Arginin, Lysin oder Ethylendiamin.

Die erfindungsgemäßen Verbindungen können in stereoisomeren Formen, entweder als Enantiomer oder als Diastereomer, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen.

Ein über N-gebundener, 3- bis 8-gliedriger gesättigter Heterocyclus, der außerdem als Heteroatome bis zu 2 Sauerstoff-, Schwefel- und/oder Stickstoffatome enthalten kann, steht im allgemeinen für Azetidinyl, Piperidyl, Morpholinyl, Piperazinyl oder Pyrrolidyl. Bevorzugt sind 5- und 6-gliedrige Ringe mit einem Sauerstoff-und/oder bis zu 2-Stickstoffatomen, wie beispielsweise Azetidinyl, Piperidyl, Morpholinyl oder Piperazinyl oder Pyrrolidinyl. Besonders bevorzugt sind Piperidyl und Pyrrolidinyl.

Bevorzugt sind Verbindungen der allgemeinen Formel (I),
in welcher

$R^1$ für geradkettiges oder verzweigtes Alkyl mit bis zu 6 Kohlenstoffatomen steht,

$R^2$ für Cyclopropyl, Cyclopentyl oder Cyclohexyl steht, oder
für geradkettiges oder verzweigtes Alkyl mit bis zu 6 Kohlenstoffatomen oder Benzyl steht, oder
für einen Rest der Formel $-CH_2CO_2R^4$ steht,
worin

$R^4$ Wasserstoff oder geradkettiges oder verzweigtes Alkyl mit bis zu 6 Kohlenstoffatomen steht,
oder

für Phenyl steht, das gegebenenfalls durch Fluor, Chlor, Brom, Cyano, Trifluormethyl oder Trifluormethoxy substituiert ist,

$R^3$ für einen Rest der Formel

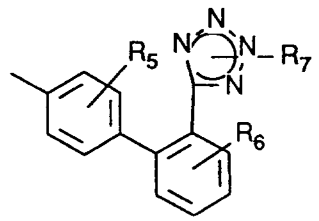

oder

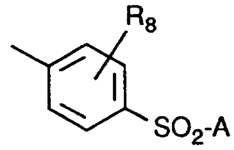

steht,
worin

$R^5$, $R^6$ und $R^8$ gleich oder verschieden sind und Wasserstoff, Fluor, Chlor, Brom oder geradkettiges oder verzweigtes Alkyl mit bis zu 4 Kohlenstoffatomen bedeuten,

$R^7$ Wasserstoff, geradkettiges oder verzweigtes Alkyl mit bis zu 4 Kohlenstoffatomen oder die Triphenylmethylgruppe bedeutet,

A ein über das Stickstoffatom gebundenes Azetidinyl, Piperidyl, Pyrrolidinyl oder Morpholinyl bedeutet, die gegebenenfalls durch einen Rest der Formel

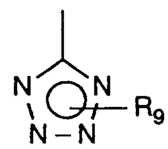

oder $-CO-R^{10}$ substituiert sind,
worin

$R^9$ die oben angegebene Bedeutung von $R^7$ hat und mit dieser gleich oder verschieden ist,

$R^{10}$ Hydroxy oder geradkettiges oder verzweigtes Alkoxy mit bis zu 4 Kohlenstoffatomen bedeutet, oder
eine Gruppe der Formel $-NR^{11}R^{12}$ bedeutet,
worin

$R^{11}$ und $R^{12}$ gleich oder verschieden sind und Wasserstoff oder geradkettiges oder verzweigtes Alkyl mit bis zu 4 Kohlenstoffatomen bedeuten,

und deren Salze.

Besonders bevorzugt sind Verbindungen der allgemeinen Formel (I),
in welcher

$R^1$ für geradkettiges oder verzweigtes Alkyl mit bis zu 5 Kohlenstoffatomen steht,

$R^2$ für Cyclopropyl, Cyclopentyl oder Cyclohexyl steht, oder
für geradkettiges oder verzweigtes Alkyl mit bis zu 5 Kohlenstoffatomen oder Benzyl steht, oder
für einen Rest der Formel $-CH_2CO_2R^4$ steht,
worin

$R^4$ Wasserstoff oder geradkettiges oder verzweigtes Alkyl mit bis zu 4 Kohlenstoffatomen bedeutet,

oder

für Phenyl steht, das gegebenenfalls durch Fluor, Chlor, Brom oder Trifluormethyl substituiert ist,

$R^3$ für einen Rest der Formel

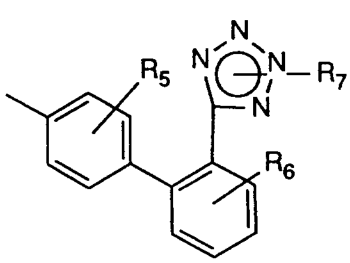

oder

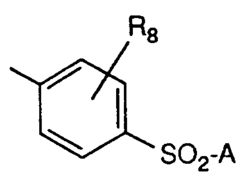

steht,

worin

| | |
|---|---|
| $R^5$, $R^6$ und $R^8$ | gleich oder verschieden sind und Wasserstoff, Fluor Chlor oder Brom bedeuten, |
| $R^7$ | Wasserstoff, Methyl, Ethyl, Propyl, Isopropyl oder die Triphenylmethylgruppe bedeutet, |
| A | über ein Stickstoffatom gebundenes Azetidinyl, Piperidyl oder Pyrrolidinyl bedeutet, die gegebenenfalls durch einen Rest der Formel |

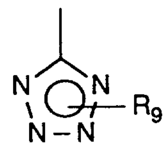

oder $-CO-R^{10}$ substituiert sind,
worin

| | |
|---|---|
| $R^9$ | die oben angegebene Bedeutung von $R^7$ hat und mit dieser gleich oder verschieden ist, |
| $R^{10}$ | Hydroxy oder geradkettiges oder verzweigtes Alkoxy mit bis zu 3 Kohlenstoffatomen bedeutet, oder eine Gruppe der Formel $-NR^{11}R^{12}$ bedeutet, worin |
| $R^{11}$ und $R^{12}$ | gleich oder verschieden sind und Wasserstoff oder geradkettiges oder verzweigtes Alkyl mit bis zu 3 Kohlenstoffatomen bedeuten, |

und deren Salze.

Außerdem wurden Verfahren zur Herstellung der erfindungsgemäßen Verbindungen der allgemeinen Formel (I) gefunden, dadurch gekennzeichnet, daß man

(I) im Fall, daß $R^3$ für den Rest der Formel

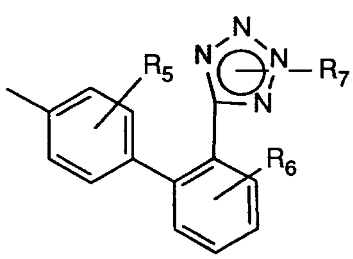

steht,
in welcher
$R^5$, $R^6$ und $R^7$ die oben angegebene Bedeutung haben
entweder
[A] Verbindungen der allgemeinen Formel (II)

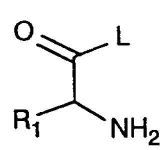

(II)

in welcher
$R^1$ die oben angegebene Bedeutung hat,
und
L für $C_1$-$C_4$-Alkoxy steht,
zunächst durch Umsetzung mit Verbindungen der allgemeinen Formel (III)

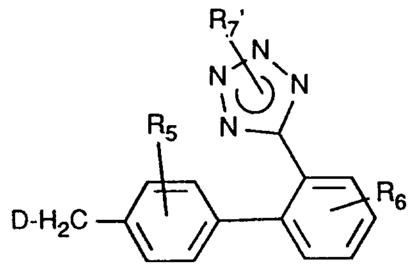

(III)

in welcher

R⁵ und R⁶ die oben angegebene Bedeutung haben,

D für Halogen, vorzugsweise für Brom steht

und

$R^{7'}$ die oben angegebene Bedeutung von $R^7$ hat aber vorzugsweise für die Triphenylmethylgruppe steht,

in inerten Lösemitteln, in Anwesenheit einer Base und eines Katalysators in die Verbindungen der allgemeinen Formel (IV)

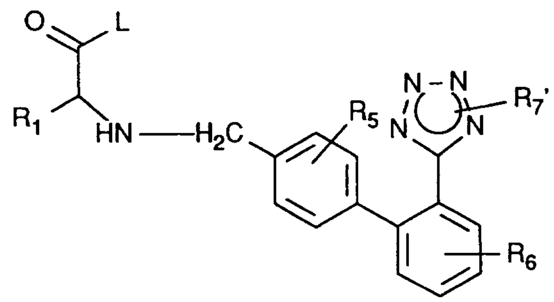

(IV)

in welcher

L, $R^1$, $R^5$, $R^6$ und $R^{7'}$ die oben angegebene Bedeutung haben,

überführt und in einem zweiten Schritt mit Verbindungen der allgemeinen Formel (V)

$R^2$-N = C = O (V)

in welcher

$R^2$ die oben angegebene Bedeutung hat,

in Anwesenheit von Säuren umsetzt,

oder

[B] Verbindungen der allgemeinen Formel (VI)

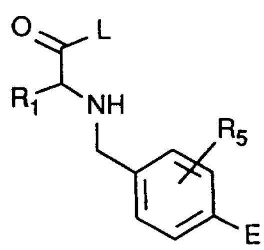

(VI)

in welcher

$R^1$, $R^2$ und L die oben angegebene Bedeutung haben

und

E für eine typische Abgangsgruppe, wie beispielsweise Brom, Jod, Methan-, Toluol-, Fluor- oder Trifluormethansulfonyloxy, vorzugsweise für Brom steht,

mit Verbindungen der allgemeinen Formel (VII)

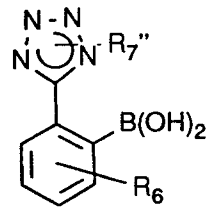

(VII)

in welcher
$R^6$ die oben angegebene Bedeutung hat
und
$R^{7''}$ für Wasserstoff oder die Triphenylmethylgruppe steht,
in inerten Lösemitteln, in Anwesenheit einer Base und metallkatalysiert umsetzt,
und
[II] im Fall, daß $R^3$ für den Rest der Formel

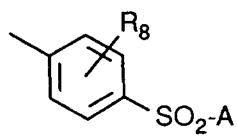

steht,
in welcher
$R^8$ und A die oben angegebene Bedeutung haben,
Verbindungen der allgemeinen Formel (II) zunächst durch Umsetzung mit Verbindungen der allgemeinen Formel (VIII)

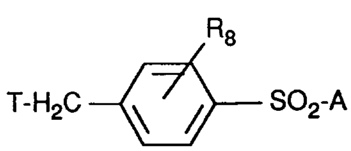

(VIII)

in welcher
A und $R^8$ die oben angegebene Bedeutung haben
und
T die oben angegebene Bedeutung von D hat und mit dieser gleich oder verschieden ist,
in inerten Lösemitteln, in Anwesenheit einer Base,
in die Verbindungen der allgemeinen Formel (IX)

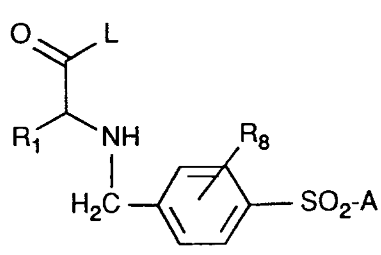

(IX)

in welcher
A, L, $R^1$ und $R^8$ die oben angegebene Bedeutung haben,
überführt und anschließend, wie unter [A] beschrieben, mit Verbindungen der allgemeinen Formel (V) umsetzt,
und anschließend in, Fall, $R^7/R^9$ = Triphenylmethylgruppe, mit Säuren in organischen Lösemitteln und/oder Wasser nach üblichen Bedingungen abspaltet,
und gegebenenfalls im Fall der unter den Substituenten $R^{10}$ aufgeführtencarbonylischen Reste, durch Verseifung der jeweiligen Ester oder durch Amidierung nach üblichen Methoden derivatisiert,

und im Fall der Salze bevorzugt ausgehend vom freien Tetrazol ($R^7/R^9$ = H) mit Säuren oder Basen umsetzt,
und gegebenenfalls auch die Substituenten $R^1$, $R^2$, $R^5$, $R^6$ und $R^8$ nach bekannten Methoden wie beispielsweise Alkylierung, Verseifung oder Amidierung, auf jeder Verfahrensstufe, variiert.

Das erfindungsgemäße Verfahren kann durch folgendes Formelschema beispielhaft erläutert werden:

[A]

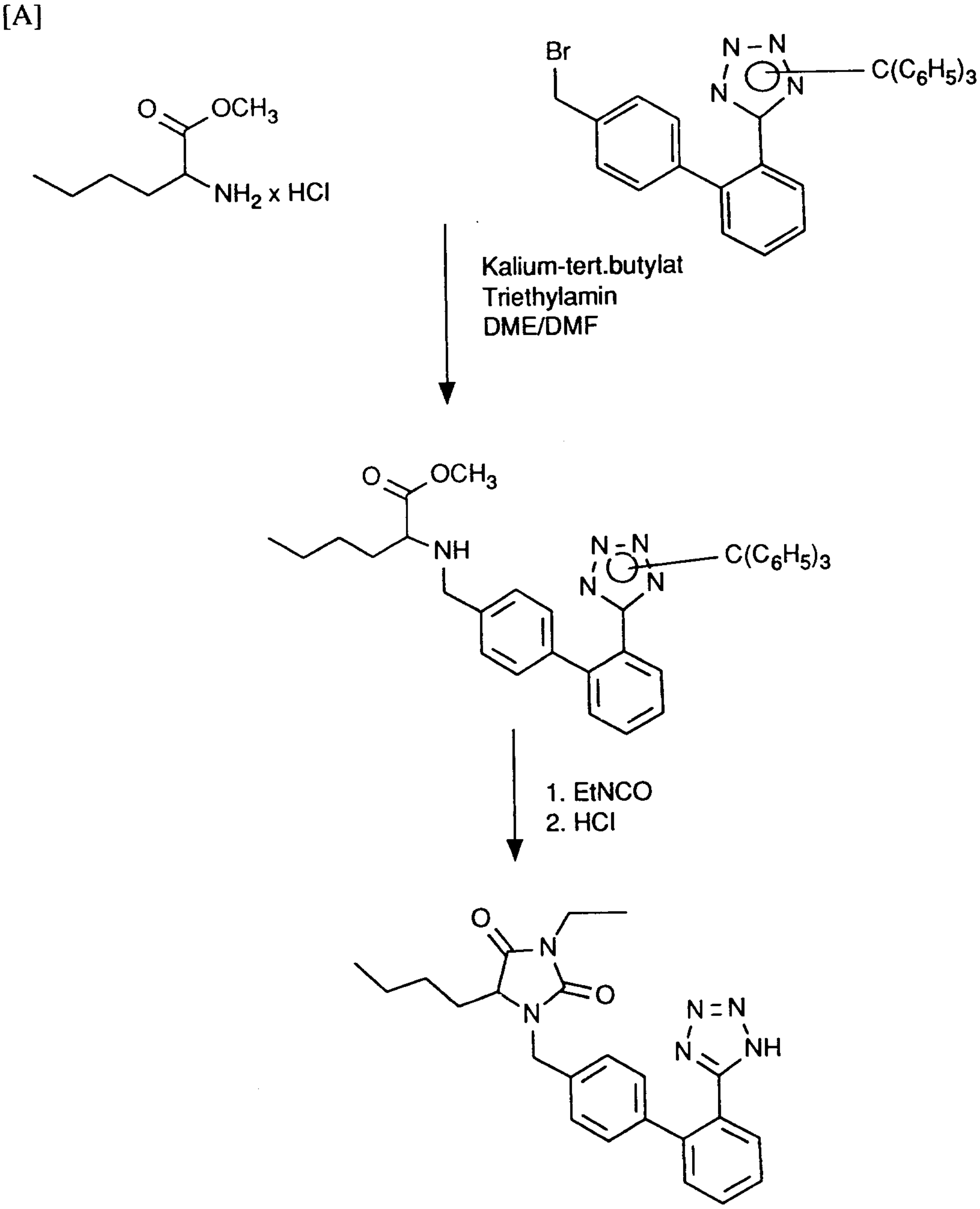

[B]

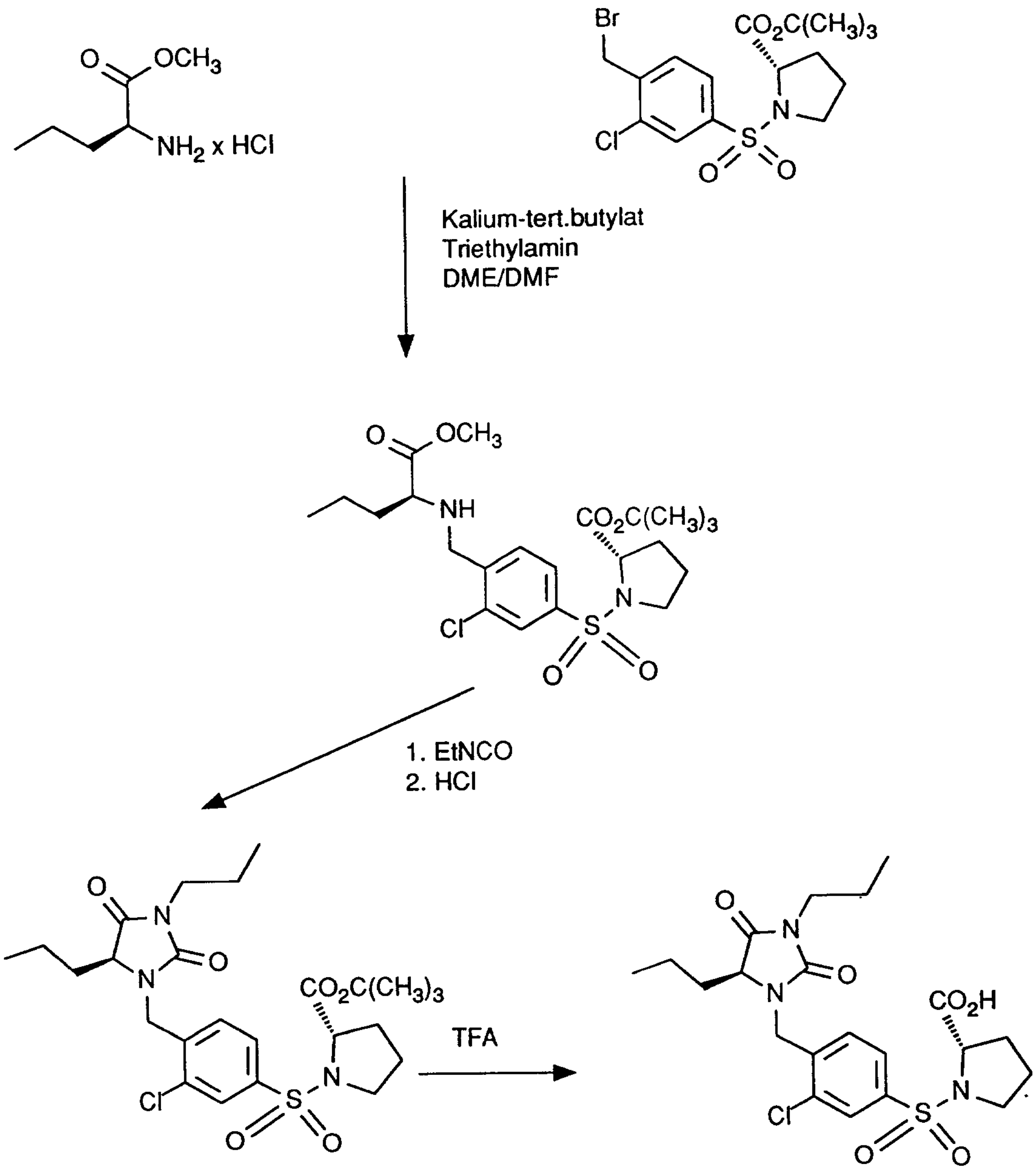


Als Lösemittel für die Verfahren [I] und [II] eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid oder Dimethoxyethan, Hexamethylphosphorsäuretriamid, Acetonitril, Aceton oder Nitromethan. Ebenso ist es möglich, Gemische der genannten Lösemittel zu verwenden. Bevorzugt für die Verfahren [I] und [II] sind Tetrahydrofuran, Aceton, Methylenchlorid, Dimethylformamid und Dimethoxyethan. Für das Verfahren [B] eignen sich außerdem noch Alkohole wie Methanol, Ethanol oder Propanol und/oder Wasser.

Als Basen für die erfindungsgemäßen Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat oder Caesiumcarbonat, oder Alkalioder Erdalkalialkoholate oder -amide wie Natrium-oder Kaliummethanolat, Natrium- oder Kaliumethanolat oder Kalium-tert.butylat, Thalliumcarbonat- oder -hydroxid, oder Lithiumdiisopropylamid (LDA), oder organi-

sche Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind für das Verfahren [A] Kaliumcarbonat, Natriumhydrid, Kalium-tert.-butylat oder Caesiumcarbonat. Bevorzugt für das Verfahren [B] ist Natriumcarbonat. Bevorzugt für das Verfahren [II] sind Natriumhydrid, Lithiumdiisopropylamid (LDA) und DBU.

Im allgemeinen setzt man die Base in einer Menge von 0,05 mol bis 10 mol, bevorzugt von 1 mol bis 2 mol jeweils bezogen auf 1 mol der Verbindungen der Formeln (III), (VI) und (VIII) ein.

Die erfindungsgemäßen Verfahren werden im allgemeinen in einem Temperaturbereich von -100 °C bis +100 °C, bevorzugt von 0 °C bis 80 °C durchgeführt. Das erfindungsgemäße Verfahren [B] erfolgt im allgemeinen unter Schutzgasatmosphäre.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton oder ebenfalls mit Alkoholen.

Die Abspaltung erfolgt im allgemeinen in einem Temperaturbereich von 0 °C bis 150 °C, vorzugsweise von 20 °C bis 100 °C und Normaldruck.

Als Katalysatoren eignen sich für das Verfahren [A] Kalium- oder Natriumiodid, bevorzugt Natriumiodid.

Als Katalysatoren für das Verfahren [B] eignen sich im allgemeinen Metallkomplexe des Nickels, Palladiums oder Platins, bevorzugt Palladium(0)-Komplexe, wie beispielsweise Tetrakistriphenylphosphinpalladium. Ebenso ist es möglich Phasen-Transfer-Katalysatoren, wie beispielsweise Tetra-n-butylammoniumbromid oder Kronenether einzusetzen.

Der Katalysator wird in einer Menge von 0,005 mol bis 0,2 mol, bevorzugt von 0,01 mol bis 0,05 mol, bezogen auf 1 mol der Verbindung der allgemeinen Formel (VI) eingesetzt.

Die Alkylierung erfolgt im allgemeinen mit Alkylierungsmitteln wie beispielsweise ($C_1$-$C_8$)-Alkylhalogeniden, Sulfonsäurestern oder substituierten oder unsubstituierten ($C_1$-$C_8$)-Dialkyl- oder ($C_1$-$C_8$)-Diarylsulfonate, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt wird Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofüran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Amidierung erfolgt im allgemeinen in einem der oben aufgeführten Lösemitteln, vorzugsweise in Tetrahydrofuran oder Dichlormethan.

Die Amidierung kann gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung erfolgt im allgemeinen in einem Temperaturbereich von - 20 ° C bis +80 ° C, vorzugsweise von -10 ° C bis +30 ° C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2 mol, bezogen auf 1 mol der entsprechenden Säure oder Esters, eingesetzt.

Die Verbindungen der allgemeinen Formeln (II),(III) und (V) sind an sich bekannt.

Die Verbindungen der allgemeinen Formel (IV) sind größtenteils neu und können wie oben beschrieben hergestellt werden.

Die Verbindungen der allgemeinen Formel (VIII) sind größtenteils neu und können beispielsweise hergestellt werden, im Fall daß $R^8 \neq$ Hydroxy oder $(C_1-C_6)$Alkoxy ($R^{8'}$) ist, indem man Verbindungen der allgemeinen Formel (X)

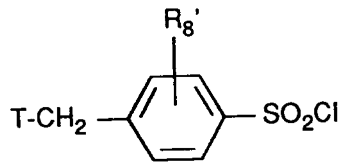

(X)

in welcher

$R^{8'}$ und T die oben angegebene Bedeutung haben,

mit Verbindungen der allgemeinen Formel (XI)

A-H (XI)

in welcher A die oben angegebene Bedeutung hat,

in einem der oben aufgeführen Lösemittel und in Anwesenheit einer der dort beschriebenen Basen, vorzugsweise in Dichlormethan/Triethylamin in einem Temperaturbereich von -10 ° C bis +120 ° C, vorzugsweise bei 0 ° C, umsetzt,

und im Fall, daß $R^8$ für Hydroxy oder $(C_1-C_6)$-Alkoxy steht,

ausgehend von Carboxy, Hydroxy-disubstituierten Benzolsulfonsäurechloriden, beispielsweise 4-Carboxy-3-hydroxybenzolsulfochlorid, zunächst durch Umsetzung mit Verbindungen der allgemeinen Formel (XI), Verbindungen der allgemeinen Formel (XII),

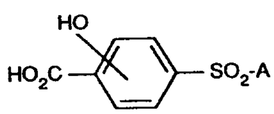

(XII)

in welcher

A die oben angegebene Bedeutung hat,

herstellt,

anschließend durch Überführung der Carboxyfunktion in den entsprechenden Benzylester und der Blockierung der Hydroxyfunktion nach üblichen Methoden in die Verbindungen der allgemeinen Formel (XIII)

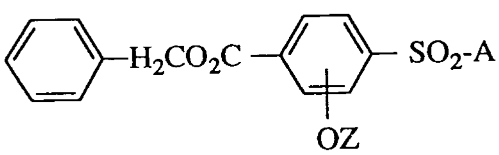

(XIII)

in welcher

A die oben angegebene Bedeutung hat

und

Z für $C_1$-$C_6$-Alkoxy steht,

überführt,

in einem weiteren Schritt, ebenfalls nach bekannten Methoden, vorzugsweise mit Natriumborhydrid/LiCl in Diglyme den Benzylester zur Hydroxymethylfunktion reduziert,

und abschließend mit Triphenylphosphindibromid, in einem der oben aufgeführten Lösemittel, vorzugsweise Dimethylformamid, unter Schutzgasatmosphäure, in einem Temperaturbereich von 0°C bis Raumtemperatur, bromiert.

Die Verbindungen der allgemeinen Formeln (X) und (XI) sind an sich bekannt.

Die Verbindungen der allgemeinen Formeln (XII) und (XIII) sind größtenteils neu und können beispielsweise wie oben beschrieben hergestellt werden.

Die Verbindungen der allgemeinen Formel (VII) mit $R^{7''}$ = H sind neu und können hergestellt werden, indem man Phenyltetrazol und Derivate zunächst unter Schutzgasatmosphäre in einem inerten Lösemittel und in Anwesenheit einer Base umsetzt und anschließend Borsäuretrimethylester zufügt und in einem letzten Schritt mit Säuren hydrolysiert.

Als Lösemittel eignen sich für das Verfahren aprotische Lösemittel wie Ether, beispielsweise Tetrahydrofuran, Diethylether, Toluol, Hexan oder Benzol. Bevorzugt ist Tetrahydrofuran.

Als Basen eignen sich sec.- und tert.Butyllithium und Phenyllithium. Bevorzugt ist n-Butyllithium.

Die Base wird in einer Menge von 2 mol bis 5 mol, bevorzugt von 2 mol bis 3 mol bezogen auf 1 mol Phenyltetrazol eingesetzt.

Als Säuren eignen sich im allgemeinen Mineralsäuren, wie beispielsweise Salzsäure, $C_1$-$C_4$-Carbonsäuren, wie beispielsweise Essigsäure oder Phosphorsäuren. Bevorzugt ist Salzsäure.

Die Säure wird im allgemeinen in einer Menge von 1 mol bis 10 mol, bevorzugt von 1 mol bis 3 mol, eingesetzt.

Das Verfahren wird im allgemeinen in einem Temperaturbereich von -70°C bis +25°C, bevorzugt von - 10°C bis 0°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formeln (IV) und (IX) sind an sich neu und können wie oben beschrieben hergestellt werden.

Die Verbindungen der allgemeinen Formel (VI) sind größtenteils neu und können beispielsweise hergestellt werden, indem man Verbindungen der allgemeinen Formel (II) mit Verbindungen der allgemeinen Formel (XIV)

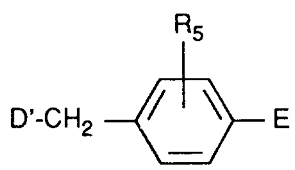

(XIV)

in welcher

$R^5$ und E die oben angegebene Bedeutung haben

und

D' die oben angegebene Bedeutung von D hat und mit dieser gleich oder verschieden ist,

in einem der oben aufgeführten Lösemittel und in Anwesenheit einer der dort beschriebenen Basen, vorzugsweise in Dimethoxyethan und Cäsiumcarbonat bei Raumtemperatur umsetzt.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen substituierten 2,4-Imidazolidindione zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimittel zur Behandlung der arteriellen Hypertonie und Atherosklerose eingesetzt werden. Darübertunaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Außerdem können die Verbindungen zur Bekämpfung von Glaukom, diabetischer Retinopathie Bewegungssteigerung der intraokularen Netzhautflüssigkeit eingesetzt werden.

Sie sind auch geeignet zur Bekämpfung von Erkrankungen des zentralen Nervensystems wie beispielsweise Depression, Migräne, Schizophrenie oder Angstzuständen, von Hirnleistungsstörungen, Schlaganfall, diabetischer Nephropathie, Herzrhytmusstörungen, Prophylaxe von koronaren Herzerkrankungen oder Restenoseprophylaxe nach Angioplastien und gefäßchrirugischen Maßnahmen.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen Applikationsvolumen pro Einzelgabe = 100 µl):

| | | |
|---|---|---|
| KCl | 22,7;32,7;42,7;52,7 | mmol/l |
| Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles

Plateau erreicht haben, werden die Testsubstanzen entweder intravenös oder als Suspension bzw. Lösung in 0,5% Tylose oral verabreicht. Die Blutdruckveränderungen unter Substanzeinfluß werden als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.

Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.

Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 $\mu$g), $^3$H-Angiotensin II (3-5 nM), Test-Pufferlösung (50 mM Tris, pH 7,2, 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 $\mu$Ci $^3$H-Thymidin zugefügt und nach weiteren 4 Stunden der Einbau dieser Substanz in die TCA-präzipitierbare DNA der Zellen bestimmt. Zur Bestimmung der $IC_{50}$-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel

als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg` vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Ausgangsverbindungen

### Beispiel I

(rac)-N-{[2'-(N'-Triphenylmethyl-tetrazol-5-yl)biphenyl-4-yl]methyl}-2-aminohexansäure-methylester

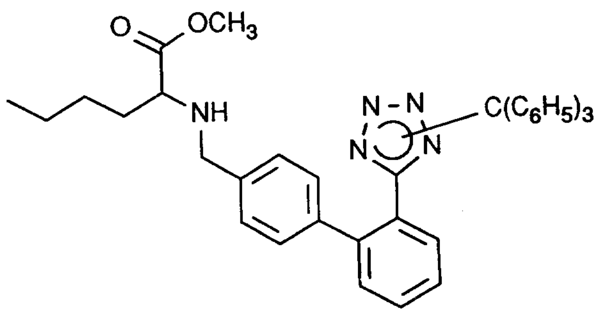

Eine Lösung von 4.08 g (22,44 mmol) (rac)-2-Aminohexansäuremethylester-hydrochlorid und 3,15 ml Triethylamin in 50 ml DME und 25 ml DMF werden unter Argon mit 2,43 g (21,69 mmol) Kalium-tert.butylat versetzt. Nach 30 min Rühren bei 20 °C wird eine Lösung von 15 g (26,93 mmol) N-Triphenylmethyl-5-[2-(4'-brommethylbiphenyl)]tetrazol in 45 ml DMF injiziert und 36 h gerührt. Anschließend wird das Solvens im Vakuum abdestilliert, der Rückstand mit Dichlormethan/Wasser aufgenommen, die organische Phase über Natriumsulfat getrocknet und nach Einengen an Kieselgel mit Petrolether/Essigester (10:1) gereinigt.
Ausbeute: 3,27 g (23% der Theorie)
$R_f$: 0,56 (Petrolether/Essigester = 3:1)

**Beispiel II**

4-[1-(S)-Methoxycarbonyl-butylamino]methyl-3-chlor-benzolsulfonyl-N-(2-(S)-tert.butoxycarbonyl)piperidinid

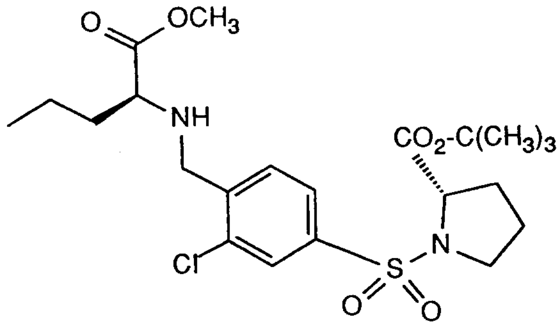

Eine Lösung von 1,68 g (10 mmol) (S)-2-Aminovaleriansäure-methylesterhydrochlorid und 1,4 ml Triethylamin in 10 ml DMF werden unter Argon mit 1,34 g (11 mmol) Kalium-tert.butylat versetzt. Nach 45 min Rühren bei 20 °C wird eine Lösung von 4,82 g (11 mmol) (S)-4-(Brommethyl)-3-chlorbenzolsulfonyl-N-2-(tert.butoxycarbonyl)pyrrolidinid in 30 ml DMF injiziert und 16 h gerührt. Anschließend wird das Solvens im Vakuum abdestilliert, der Rückstand mit Dichlormethan/Wasser aufgenommen, die organische Phase über Natriumsulfat getrocknet und nach Einengen an Kieselgel mit Petrolether/Essigester (10:1) gereinigt.
Ausbeute: 1,8 g (37% der Theorie)
$R_f$: 0,76 (Petrolether/Essigester = 2:1)

**Beispiel III**

4-[(3S,5-Dipropylimidazolidin-2,4-dion-1-yl]methyl-3-chlor-benzolsulfonyl-N-(2-S-tert.butoxycarbonyl)-piperidinid

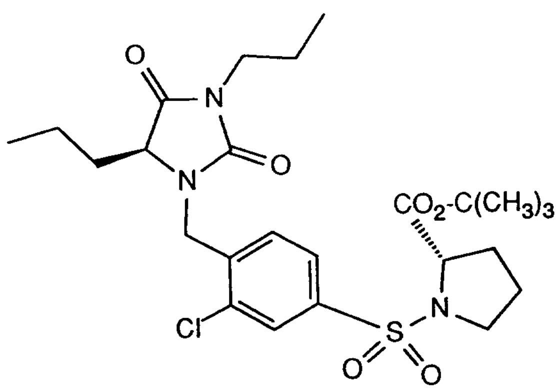

Eine lösung von 489 mg (1,0 mmol) der Verbindung aus Beispiel II und 255 mg (3,0 mmol) Propylisocyanat in 10 ml Essigester werden 16 h am Rückfluß erhitzt. Anschließend werden 10 ml Essigester und 5 ml 5%ige Salzsäure zugegeben und mit je 10 ml Wasser, ges. Natriumhydrogencarbonat und ges. Kochsalz-Lösung gewaschen. Die organische Phase wird abgetrennt, über Natriumsulfat getrocknet, eingeengt und an Kieselgel mit Petrolether/Essigester (4:1) gereinigt.
Ausbeute: 390 mg (68% der Theorie)
$R_f$ = 0,63 (Petrolether/Essigester = 2:1)

## Herstellungsbeispiele

### Beispiel 1

(rac)-5-Buty-3-ethyl-1-{[2'-(tetrazol-5-yl)biphenyl-4-yl]methyl}-imidazolidin-2,4-dion

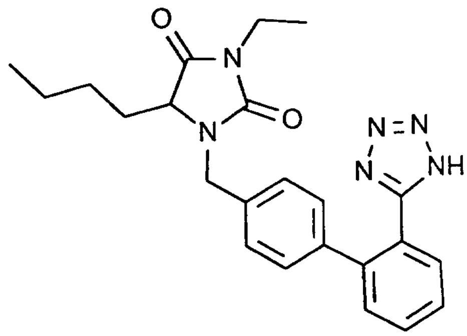

Eine Lösung von 300 mg (0,48 mmol) der Verbindung aus Beispiel 1 und 0,115 ml (1,45 mmol) Ethylisocyanat in 10 ml Essigester werden 16 h am Rückfluß erhitzt. Anschließend gibt man 2 ml halbkonzentrierte Salzsäure in der Siedehitze hinzu und entfernt das Heizbad. Nach 1 h wird die erkaltete Lösung mit Dichlormethan verdünnt, die Phasen werden getrennt und die wäßrige Phase zweimal mit je 20 ml Dichlormethan/Essigester (1:1) extrahiert. Die vereinigten organischen Phasen werden über Natriumsulfat getrocknet, das Solvens entfernt und der Rückstand an Kieselgel mit Toluol/Essigester/Eisessig (35:5:1) gereinigt.
Ausbeute: 65,4 mg (32% der Theorie)
$R_f$: 0,37 (Toluol/Essigester/Eisessig = 20:20:1)
In Analogie zur Vorschrift des Beispiels 1 werden die in den Tabellen 1 und 2 aufgeführten Verbindungen hergestellt:

Tabelle 1:

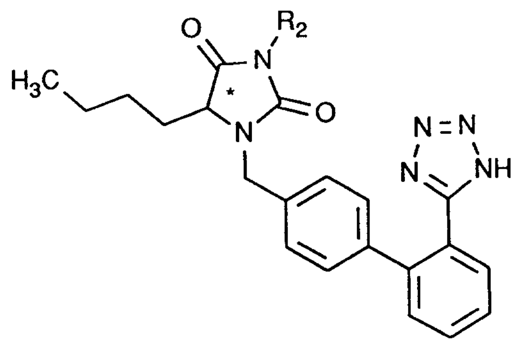

| Bsp.-Nr. | * | $R^2$ | $R_f$ (Laufmittel A) |
|---|---|---|---|
| 2 | (R) | $-C_2H_5$ | 0,37 |
| 3 | (S) | $-C_2H_5$ | 0,37 |
| 4 | (R) | $-(CH_2)_2-CH_3$ | 0,43 |
| 5 | (S) | $-(CH_2)_2-CH_3$ | 0,43 |
| 6 | (rac) | $-(CH_2)_2-CH_3$ | 0,43 |
| 7 | (R) | $-(CH_2)_3-CH_3$ | 0,48 |
| 8 | (S) | $-(CH_2)_3-CH_3$ | 0,48 |
| 9 | (rac) | $-(CH_2)_3-CH_3$ | 0,48 |
| 10 | (R) | $-CH(CH_3)_2$ | 0,57 |
| 11 | (S) | $-CH(CH_3)_2$ | 0,57 |
| 12 | (rac) | $-CH(CH_3)_2$ | 0,57 |
| 13 | (R) | $-C_6H_{11}$ | 0,55 |
| 14 | (S) | $-C_6H_{11}$ | 0,55 |
| 15 | (rac) | $-C_6H_{11}$ | 0,55 |
| 16 | (R) | $-CH_2-C_6H_5$ | 0,53 |
| 17 | (S) | $-CH_2-C_6H_5$ | 0,53 |
| 18 | (rac) | $-CH_2-C_6H_5$ | 0,53 |

Fortsetzung Tabelle 1:

| Bsp.-Nr. | * | $R^2$ | $R_f$ (Laufmittel A) |
|---|---|---|---|
| 19 | (R) | $-C_6H_5$ | 0,52 |
| 20 | (S) | $-C_6H_5$ | 0,52 |
| 21 | (rac) | $-C_6H_5$ | 0,52 |
| 22 | (R) | $-C_6H_4$-o-$CF_3$ | 0,57, 0.49 |
| 23 | (S) | $-C_6H_4$-o-$CF_3$ | 0,57, 0,49 |
| 24 | (rac) | $-C_6H_4$-o-$CF_3$ | 0,57, 0,49 |
| 25 | (R) | $-CH_2CO_2C_2H_5$ | 0,46 |
| 26 | (S) | $-CH_2CO_2C_2H_5$ | 0,46 |
| 27 | (rac) | $-CH_2-CO_2C_2H_5$ | 0,46 |
| 28 | (R) | $-CH_2CO_2H$ | 0,11 |
| 29 | (rac) | $-CH_2CO_2H$ | 0,11 |

Laufmittel A : Toluol / Essigester / Eisessig (20:20:1)

Tabelle 2:

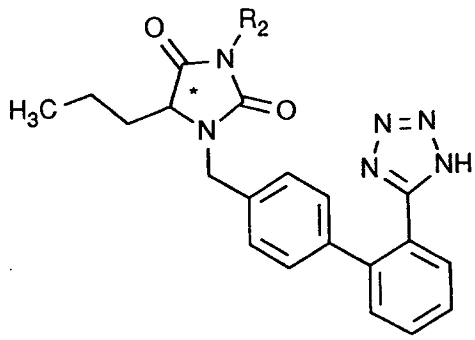

| Bsp.-Nr. | * | $R^2$ | $R_f$ (Laufmittel A) |
|---|---|---|---|
| 30 | (R) | $-C_2H_5$ | 0,17 |
| 31 | (S) | $-C_2H_5$ | 0,17 |
| 32 | (rac) | $-C_2H_5$ | 0,17 |
| 33 | (R) | $-(CH_2)_2CH_3$ | 0,20 |
| 34 | (S) | $-(CH_2)_2CH_3$ | 0,20 |
| 35 | (rac) | $-(CH_2)_2CH_3$ | 0,20 |
| 36 | (R) | $-(CH_2)_3CH_3$ | 0,25 |
| 37 | (S) | $-(CH_2)_3CH_3$ | 0,25 |
| 38 | (rac) | $-(CH_2)_3CH_3$ | 0,25 |
| 39 | (R) | $-CH(CH_3)_2$ | 0,34 |
| 40 | (S) | $-CH(CH_3)_2$ | 0,34 |
| 41 | (rac) | $-CH(CH_3)_2$ | 0,34 |
| 42 | (R) | $-C_6H_{11}$ | 0,30 |
| 43 | (S) | $-C_6H_{11}$ | 0,30 |
| 44 | (rac) | $-C_6H_{11}$ | 0,30 |
| 45 | (R) | $-CH_2C_6H_5$ | 0,54 |
| 46 | (S) | $-CH_2C_6H_5$ | 0,54 |

Fortsetzung Tabelle 2:

| Bsp.-Nr. | * | $R^2$ | $R_f$ (Laufmittel A) |
|---|---|---|---|
| 47 | (rac) | $-CH_2-C_6H_5$ | 0,54 |
| 48 | (R) | $-C_6H_5$ | 0,27 |
| 49 | (S) | $-C_6H_5$ | 0,27 |
| 50 | (rac) | $-C_6H_5$ | 0,27 |
| 51 | (R) | $-C_6H_4-o-CF_3$ | 0,26, 0.17 |
| 52 | (S) | $-C_6H_4-o-CF_3$ | 0,26, 0,17 |
| 53 | (rac) | $-C_6H_4-o-CF_3$ | 0,26, 0,17 |
| 54 | (R) | $-CH_2CO_2C_2H_5$ | 0,69 |
| 55 | (S) | $-CH_2CO_2C_2H_5$ | 0,69 |
| 56 | (rac) | $-CH_2-CO_2C_2H_5$ | 0,69 |
| 57 | (R) | $-CH_2CO_2H$ | 0,18 |
| 58 | (S) | $-CH_2CO_2H$ | 0,18 |
| 59 | (rac) | $-CH_2CO_2H$ | 0,18 |

Laufmittel A: Toluol / Essigester / Eisessig (30:10:1)

Beispiel 60

4-[(3S,5-Dipropylimidazolidin-2,4-dion-1-yl]methyl-3-chlor-benzolsulfonyl-N-(2-S-carboxy)piperidinid

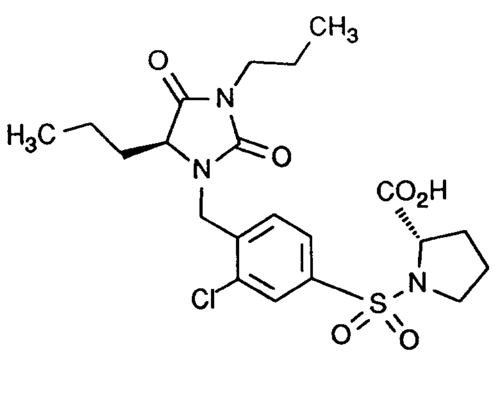

Zu einer Lösung von 360 mg (0,66 mmol) der Verbindung aus Beispiel III in 3,5 ml Dichlormethan werden 1,45 ml Trifluoressigsäure gegeben und 2 h bei 20°C gerührt. Anschließend wird mit 10 ml

Dichlormethan verdünnt, mit 10 ml Wasser gewaschen, die organische Phase nach Abtrennung über Natriumsulfat getrocknet und eingeengt. Der Rückstand wird an Kieselgel mit Toluol/Essigester/Eisessig (35:5:1) gereinigt.

Ausbeute: 252 mg (78% der Theorie)

$R_f$ = 0,27 (Toluol/Essigester/Eisessig 30:10:1)

In Analogie zur Vorschrift des Beispiels 60 werden die in Tabelle 3 aufgeführten Verbindungen hergestellt:

Tabelle 3:

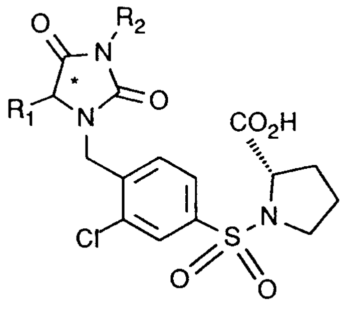

| Bsp.-Nr. | * | $R^1$ | $R^2$ | $R_f$ (Laufmittel A) |
|---|---|---|---|---|
| 61 | (rac) | $-(CH_2)_2CH_3$ | $-C_2H_5$ | 0,25 |
| 62 | (S) | $-(CH_2)_2CH_3$ | $-C_2H_5$ | 0,25 |
| 63 | (S) | $-(CH_2)_2CH_3$ | $-(CH_2)_2CH_3$ | 0,27 |
| 64 | (S) | $-(CH_2)_2CH_3$ | $-(CH_2)_3CH_3$ | 0,27 |
| 65 | (S) | $-(CH_2)_2CH_3$ | $-CH_2CO_2C_2H_5$ | 0,25 |
| 66 | (S) | $-(CH_2)_3CH_3$ | $-(CH_2)_2CH_3$ | 0,22 |
| 67 | (S) | $-(CH_2)_3CH_3$ | $-(CH_2)_3CH_3$ | 0,24 |
| 68 | (S) | $-(CH_2)_3CH_3$ | $-CH_2CO_2C_2H_5$ | 0,19 |
| 69 | (S) | $-CH(CH_3)_2$ | $-C_2H_5$ | 0,2 |
| 70 | (S) | $-CH(CH_3)_2$ | $-(CH_2)_2CH_3$ | 0,2 |
| 71 | (S) | $-CH(CH_3)_2$ | $-(CH_2)_3CH_3$ | 0,36 |

Laufmittel A: Toluol/Essigester/Eisessig (30:10:1)

**Patentansprüche**

1. Substituierte 2,4-Imidazolidindione der allgemeinen Formel

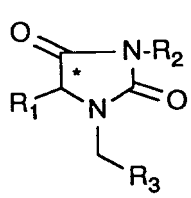

(I)

in welcher

$R^1$ für geradkettiges oder verzweigtes Alkyl mit bis zu 8 Kohlenstoffatomen steht,

$R^2$ für Cycloalkyl mit 3 bis 8 Kohlenstoffatomen steht, oder für geradkettiges oder verzweigtes Alkyl mit bis zu 8 Kohlenstoffatomen steht, das gegebenenfalls durch Phenyl substituiert ist, oder für eine Gruppe der Formel $-CH_2-CO_2R^4$ steht, worin

$R^4$ Wasserstoff oder geradkettiges oder verzweigtes Alkyl mit bis zu 8 Kohlenstoffatomen bedeutet,

oder

für Phenyl steht, das gegebenenfalls durch Halogen, Cyano, Trifluormethyl oder Trifluormethoxy substituiert ist,

$R^3$ für einen Rest der Formel

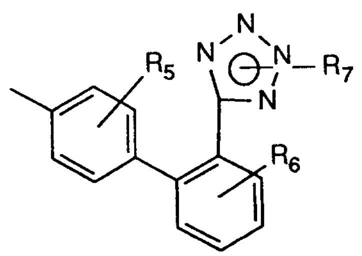

oder

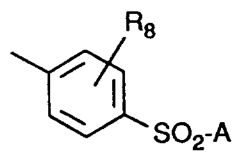

steht,

worin

$R^5$, $R^6$ und $R^8$ gleich oder verschieden sind und Wasserstoff, Halogen, Cyano, Nitro oder für geradkettiges oder verzweigtes Alkyl oder Alkoxy mit jeweils bis zu 6 Kohlenstoffatomen bedeuten,

$R^7$ Wasserstoff, geradkettiges oder verzweigtes Alkyl mit bis zu 6 Kohlenstoffatomen oder die Triphenylmethylgruppe bedeutet,

A einen über das Stickstoffatom gebundenen 3 bis 8-gliedrigen, gesättigten Heterocyclus mit bis zu 2 Heteroatomen aus der Reihe S, N oder O bedeutet und der gegebenenfalls bis zu 2-fach gleich oder verschieden durch einen Rest der Formel

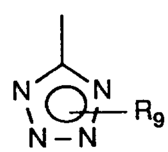

oder $-CO-R^{10}$ substituiert ist,

worin

$R^9$ die oben angegebene Bedeutung von $R^7$ hat und mit dieser gleich oder verschieden ist,

$R^{10}$ Hydroxy oder geradkettiges oder verzweigtes Alkoxy mit bis zu 6 Kohlenstoffatomen bedeutet, oder eine Gruppe der Formel $-NR^{11}R^{12}$ bedeutet, worin

$R^{11}$ und $R^{12}$ gleich oder verschieden sind und Wasserstoff oder geradkettiges oder verzweigtes Alkyl mit bis zu 6 Kohlenstoffatomen bedeuten

und deren Salze.

**2.** Substituierte 2,4-Imidazolidindione nach Anspruch 1 worin in welcher

$R^1$ für geradkettiges oder verzweigtes Alkyl mit bis zu 6 Kohlenstoffatomen steht,

$R^2$ für Cyclopropyl, Cyclopentyl oder Cyclohexyl steht, oder
für geradkettiges oder verzweigtes Alkyl mit bis zu 6 Kohlenstoffatomen oder Benzyl steht, oder
für einen Rest der Formel $-CH_2CO_2R^4$ steht,
worin
$R^4$ Wasserstoff oder geradkettiges oder verzweigtes Alkyl mit bis zu 6 Kohlenstoffatomen steht,
oder
für Phenyl steht, das gegebenenfalls durch Fluor, Chlor, Brom, Cyano, Trifluormethyl oder Trifluormethoxy substituiert ist,
$R^3$ für einen Rest der Formel

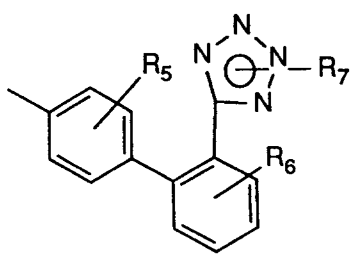

oder

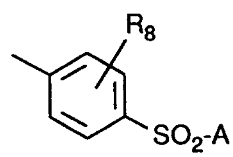

steht,
worin
$R^5$, $R^6$ und $R^8$ gleich oder verschieden sind und
Wasserstoff, Fluor, Chlor, Brom oder geradkettiges oder verzweigtes Alkyl mit bis zu 4 Kohlenstoffatomen bedeuten,
$R^7$ Wasserstoff, geradkettiges oder verzweigtes Alkyl mit bis zu 4 Kohlenstoffatomen oder die Triphenylmethylgruppe bedeutet,
A ein über das Stickstoffatom gebundenes Azetidinyl, Piperidyl, Pyrrolidinyl oder Morpholinyl bedeutet, die gegebenenfalls durch einen Rest der Formel

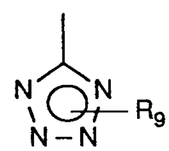

oder $-CO-R^{10}$ substituiert sind,
worin
$R^9$ die oben angegebene Bedeutung von $R^7$ hat und mit dieser gleich oder verschieden ist,
$R^{10}$ Hydroxy oder geradkettiges oder verzweigtes Alkoxy mit bis zu 4 Kohlenstoffatomen bedeutet, oder
eine Gruppe der Formel $-NR^{11}R^{12}$ bedeutet,
worin
$R^{11}$ und $R^{12}$ gleich oder verschieden sind und Wasserstoff oder geradkettiges oder verzweigtes Alkyl mit bis zu 4 Kohlenstoffatomen bedeuten,
und deren Salze.

**3.** 2,4-Imidazolidindione nach Anspruch 1
worin
$R^1$ für geradkettiges oder verzweigtes Alkyl mit bis zu 5 Kohlenstoffatomen steht,
$R^2$ für Cyclopropyl, Cyclopentyl oder Cyclohexyl steht, oder
für geradkettiges oder verzweigtes Alkyl mit bis zu 5 Kohlenstoffatomen oder Benzyl steht, oder
für einen Rest der Formel $-CH_2CO_2R^4$ steht,
worin
$R^4$ Wasserstoff oder geradkettiges oder verzweigtes Alkyl mit bis zu 4 Kohlenstoffatomen

bedeutet,

oder

für Phenyl steht, das gegebenenfalls durch Fluor, Chlor, Brom oder Trifluormethyl substituiert ist,

$R^3$ für einen Rest der Formel

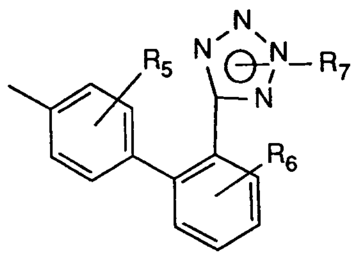

oder

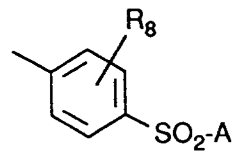

steht,

worin

$R^5$, $R^6$ und $R^8$ gleich oder verschieden sind und Wasserstoff, Fluor, Chlor oder Brom bedeuten,

$R^7$ Wasserstoff, Methyl, Ethyl, Propyl, Isopropyl oder die Triphenylmethylgruppe bedeutet,

A über ein Stickstoffatom gebundenes Azetidinyl, Piperidyl oder Pyrrolidinyl bedeutet, die gegebenenfalls durch einen Rest der Formel

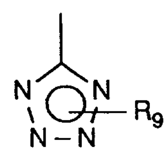

oder $-CO-R^{10}$ substituiert sind,

worin

$R^9$ die oben angegebene Bedeutung von $R^7$ hat und mit dieser gleich oder verschieden ist,

$R^{10}$ Hydroxy oder geradkettiges oder verzweigtes Alkoxy mit bis zu 3 Kohlenstoffatomen bedeutet, oder

eine Gruppe der Formel $-NR^{11}R^{12}$ bedeutet,

worin

$R^{11}$ und $R^{12}$ gleich oder verschieden sind und Wasserstoff oder geradkettiges oder verzweigtes Alkyl mit bis zu 3 Kohlenstoffatomen bedeuten,

und deren Salze.

**4.** Substituierte 2,4-Imidazolidindione nach Ansprüchen 1-3 zur therapeutischen Anwendung.

**5.** Verfahren zur Herstellung von substituierten 2,4-Imidazolidindionen nach Ansprüchen 1-3. dadurch gekennzeichnet, daß man

(I) im Fall, daß $R^3$ für den Rest der Formel

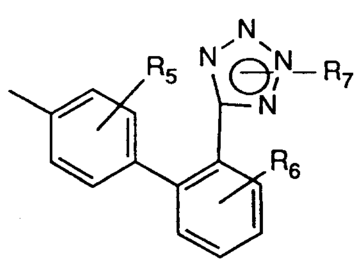

steht,

in welcher

$R^5$, $R^6$ und $R^7$ die oben angegebene Bedeutung haben

entweder

[A] Verbindungen der allgemeinen Formel (II)

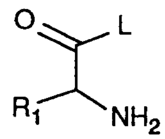

(II)

in welcher

$R^1$ die oben angegebene Bedeutung hat,

und

L für $C_1$-$C_4$-Alkoxy steht,

zunächst durch Umsetzung mit Verbindungen der allgemeinen Formel (III)

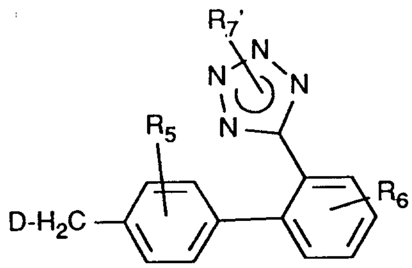

(III)

in welcher

$R^5$ und $R^6$ die oben angegebene Bedeutung haben,

D für Halogen, vorzugsweise für Brom steht

und

$R^{7'}$ die oben angegebene Bedeutung von $R^7$ hat, aber vorzugsweise für die Triphenylmethylgruppe steht,

in inerten Lösemitteln, in Anwesenheit einer Base und eines Katalysators in die Verbindungen der allgemeinen Formel (IV)

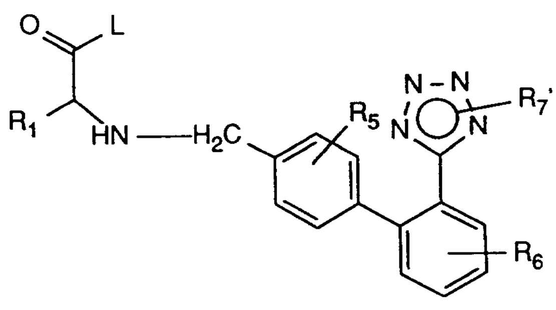

(IV)

in welcher

L, $R^1$, $R^5$, $R^6$ und $R^{7'}$ die oben angegebene Bedeutung haben,

überführt und in einem zweiten Schritt mit Verbindungen der allgemeinen Formel (V)

$R^2$-N=C=O (V)

in welcher

$R^2$ die oben angegebene Bedeutung hat,

in Anwesenheit von Säuren umsetzt,

oder

[B] Verbindungen der allgemeinen Formel (VI)

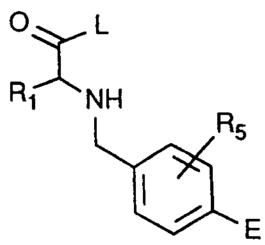

(VI)

in welcher

$R^1$, $R^2$ und L die oben angegebene Bedeutung haben

und

E für eine typische Abgangsgruppe, wie beispielsweise Brom, Jod, Methan-, Toluol-, Fluor- oder Trifluormethansulfonyloxy, vorzugsweise für Brom steht,

mit Verbindungen der allgemeinen Formel (VII)

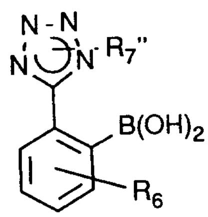

(VII)

in welcher

$R^6$ die oben angegebene Bedeutung hat

und

$R^{7''}$ für Wasserstoff oder die Triphenylmethylgruppe steht,

in inerten Lösemitteln, in Anwesenheit einer Base und metallkatalysiert umsetzt,

und

[II] im Fall, daß $R^3$ für den Rest der Formel

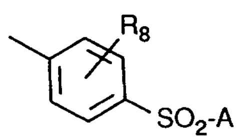

steht,

in welcher

$R^8$ und A die oben angegebene Bedeutung haben,

Verbindungen der allgemeinen Formel (II) zunächst durch Umsetzung mit Verbindungen der allgemeinen Formel (VIII)

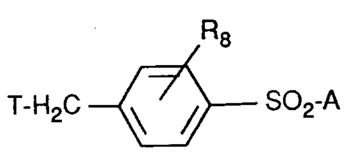

(VIII)

in welcher

A und $R^8$ die oben angegebene Bedeutung haben

und

T die oben angegebene Bedeutung von D hat und mit dieser gleich oder verschieden ist,

in inerten Lösemitteln, in Anwesenheit einer Base,

in die Verbindungen der allgemeinen Formel (IX)

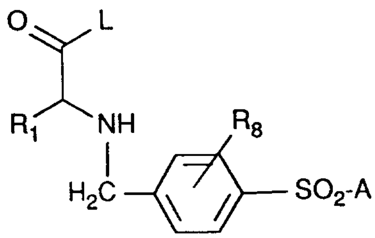

(IX)

in welcher

A, L, $R^1$ und $R^8$ die oben angegebene Bedeutung haben,

überführt und anschließend, wie unter [A] beschrieben, mit Verbindungen der allgemeinen Formel (V) umsetzt,

und anschließend im Fall, $R^7/R^9$ = Triphenylmethylgruppe, mit Säuren in organischen Lösemitteln und/oder Wasser nach üblichen Bedingungen abspaltet,

und gegebenenfalls im Fall der unter den Substituenten $R^{10}$ aufgeführten carbonylischen Reste, durch Verseifung der jeweiligen Ester oder durch Amidierung nach üblichen Methoden derivatisiert,

und im Fall der Salze bevorzugt ausgehend vom freien Tetrazol ($R^7/R^9$ = H) mit Säuren oder Basen umsetzt,

und gegebenenfalls auch die Substituenten $R^1$, $R^2$, $R^5$, $R^6$ und $R^8$ nach bekannten Methoden wie beispielsweise Alkylierung, Verseifung oder Amidierung, auf jeder Verfahrensstufe, variiert.

**6.** Arzneimittel enthaltend substituierte 2,4-Imidazolidindione nach Ansprüchen 1-3.

**7.** Arzneimittel nach Anspruch 6 zur Behandlung von arterieller Hypertonie und Arteriosklerose.

**8.** Verfahren zur Herstellung von Arzneimitteln nach Ansprüchen 6 und 7, dadurch gekennzeichnet, daß man die substituierten 2,4-Imidazolidindione gegebenenfalls unter Verwendung von geeigneten Hilfs- und Trägerstoffen in eine geeignete Applikationsform überführt.

**9.** Verwendung von 2,4-Imidazolidindionen nach Ansprüchen 1-3 zur Herstellung von Arzneimitteln.

**10.** Verwendung nach Anspruch 9 zur Herstellung von Arzneimitteln zur Behandlung von arterieller Hypertonie und Arteriosklerose.

Europäisches Patentamt

# EUROPÄISCHER RECHERCHENBERICHT

Nummer der Anmeldung
EP 94 10 7474

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X<br><br>Y | WO-A-92 07834 (G.D. SEARLE & CO.)<br>* Seite 233 - Seite 238; Anspruch 1 *<br>* Seite 259 - Seite 261; Anspruch 8 *<br>--- | 1-4,6-10<br><br>1-4,9,10 | C07D403/10<br>C07D403/12<br>A61K31/415<br>A61K31/41<br>A61K31/40 |
| Y | EP-A-0 324 377 (E.I. DU PONT DE NEMOURS AND COMPANY)<br>* Seite 251 - Seite 259; Anspruch 9; darin im besonderen: Seite 252, Zeile 11 und Zeilen 43-47 *<br>* Seite 102; Beispiel 83; Tabelle 5 *<br>--- | 1-4,9,10 | |
| Y | EP-A-0 412 594 (MERCK & CO. INC.)<br>* Seite 62 - Seite 74; Ansprüche 1-8 *<br>* Seite 41 - Seite 44; Beispiele 3-5 *<br>* Seite 49; Tabelle I, Verbindung Nr. (1) *<br>* Seite 57; Tabelle I, Verbindungen Nr. (44) und (45) *<br>* Seite 3, Zeile 37 - Zeile 39 *<br>--- | 1-4,9,10 | |
| D,Y | CHEMICAL ABSTRACTS, vol. 88, no. 7, 13. Februar 1978, Columbus, Ohio, US; abstract no. 50859z, C. TASHIRO 'Hydantoins' Seite 549 ;Spalte 1 ;<br>* Zusammenfassung; und Chemical Abstracts, CHEMICAL SUBSTANCES, Tenth Collective Index, Vol. 86-95, 1977-1981, Seite 26538CS und RN [65346-93-2] *<br>& JP-A-77 100 469 (YOSHITOMI PHARMACEUTICAL INDUSTRIES, LTD.) 23. August 1977<br>----- | 1-4,9,10 | RECHERCHIERTE SACHGEBIETE (Int.Cl.5)<br>C07D |

Der vorliegende Recherchenbericht wurde für alle Patentansprüche erstellt

| Recherchenort | Abschlußdatum der Recherche | Prüfer |
|---|---|---|
| DEN HAAG | 30. Juni 1994 | Fink, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)